# EUROPEAN PATENT APPLICATION

(11) **EP 1 489 101 A1**
(43) Date of publication of application: **22.12.2004**
(21) Application number: 03743526.0
(22) Date of filing: 28.02.2003
(51) Int. Cl.: C08B 37/00, A61K 31/715, A61P 31/16

(54) **NOVEL BRANCHED SIALO-SUGAR MOLECULES AND ANTIVIRAL AGENTS USING THE SAME**

(30) Priority: 04.03.2002 JP 2002057909
(71) Applicant: Japan Science and Technology Agency, Kawaguchi-shi, Saitama 332-0012 (JP)
(72) Inventor: SUZUKI, Yasuo, Shizuoka-shi, Shizuoka 420-0911 (JP); JWA, Ilpal, Shimizu-shi, Shizuoka 424-0857 (JP)
(74) Representative: Owen, Deborah Jane
(86) International application number: PCT/JP2003/002338
(87) International publication number: WO 2003/074570

(57) **Abstract**

A novel branched sialo-sugar molecule represented by the following formula (I) which is a substance which responds to a variation in the host range of an influenza virus or a variation in antigenicity and is useful as a drug or an adsorbent in a virus removal filter or the like capable of preventing the infection with a type A influenza virus and a type B influenza virus originating in any animals including humans: 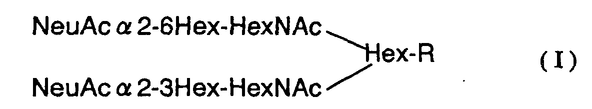 (wherein NeuAc represents *N*-acetylneuraminic acid in which the hydroxyl group, the carboxyl group and the amido group may be chemically modified with a halogen group, an alkyl group or an acyl group, either the same group or separate groups, Hex represents hexose, HexNAc represents N-acetylhexosamine and R represents a substrate selected from among a hydrogen atom, a hydrocarbon chain, a sugar chain, a lipid, a protein and a synthetic polymer, R may have a substituent, and the linkage between *N*-acetylneuraminic acid and hexose may be either an O-glycoside linkage occurring in nature or a chemically converted linkage such as an S-glycoside or a Se-glycoside linkage) is provided.

## Description

### Technical Field

The invention of this application relates to a novel branched sialo-sugar molecule. More particularly, the invention of this application relates to a novel branched sialo-sugar molecule which can respond to a variation in the host range of an influenza virus or a variation in antigenicity and is useful as a drug or an adsorbent in a virus removal filter or the like capable of preventing infection with a type A influenza virus and a type B and thus preventing a flu epidemic.

### Background Art

The number of patients infected with influenza in Japan is from several 100 thousands to one million every year. The Spanish flu in 1918 (it was found that the origin was H1N1 influenza virus by gene analysis) killed at least 20 million people worldwide a year, including more than 380 thousand people in Japan, which has been ranked as the worst natural disaster in recorded history.

In addition, it has been reported recently that particularly for elderly people flu causes complications of bronchitis or pneumonia and so becomes a critical disease, and that it causes encephalitis or encephalopathy in children, and therefore a cure for the disease in winter has become an urgent need worldwide.

The influenza pathogen is an influenza virus, and three types: type A, B and C, based on the difference in antigenicities of the internal protein, are known. Type B and C viruses are mainly isolated from humans; however, type A virus is isolated from many species of animals in nature such as wild aquatic birds, domestic poultry, pigs, horses, seals, whales and ferrets other than humans, and its pathogenicity is the highest, and causes worldwide epidemics.

The genome of influenza virus consists of single strand RNA segments. It is known that generally the replication by RNA polymerase that reads sequences of a single strand RNA is far more likely to be in error than the replication by DNA polymerase that reads a common double strand DNA (Steinhauer, D.A., Holland, J.J.: *Annual Reviw of Microbiology,* 41, 409-433 (1987): Gojobori, T., Yamaguchi, Y., Ikeo, K., Mizokami, M.: *Japanese Journal of Genetics,* 69, 481-488 (1994)). Therefore, when a virus is grown in a host cell, a new variant virus is generated at the same time. Due to such a variation, the antigenicity of a virus protein changes gradually, therefore, there are many cases where influenza viruses are not detected by a common immunological surveillance system, thereby causing a flu epidemic every year.

Variations of influenza viruses include, other than a series of antigenic variations (Antigenic Drift) described above, a variation in the host range (Antigenic Shift) (Suzuki, Y.: *Progress in Lipid Research,* 33, 429-457 (1994)). The genes of an influenza virus are not united into one, but are segments, therefore, if, for example, a pig is infected with human and duck influenza viruses simultaneously, a rearrangement of both genes may be generated in the body of the pig whereby a virus having a completely new type (antigenicity) may be generated. Meanwhile, humans do not have any antibody at all against such a virus; therefore, if humans are infected with such a new virus, there is a risk of an outbreak of a flu epidemic worldwide.

It is known that infection with influenza virus is caused by binding of hemagglutinin (HA) that is a viral glycoprotein to a sialic acid-containing sugar chain present on the surface of a host cell (Suzuki, Y.: *Progress in Lipid Research,* 33, 429-457 (1994): Paulson, J.C.: The Receptors, Vol. 2. Academic Press, Orlando, pp. 131-219 (1985): Wiley, D.C. and Skehel, J.J.: *Annual Review of Biochemistry,* 56, 365-394 (1987)). Therefore, conventionally, this sialic acid-containing sugar chain has been studied, and various sialic acid-containing sugar chain (sialo-sugar chain) compounds as an anti-influenza virus agent have been reported (Fujimoto, K., Hayashida, O., Aoyama, Y., Guo, C.T., Hidari, K.I.P.J., and Suzuki, Y.: *Chemistry Letter,* 1259-1260 (1999); Suzuki, Y., Sato, K., Kiso, M., and Hasegawa, A.: *Glycoconj J.* 7, 349-54 (1990)). However, all of these compounds show an anti-influenza virus activity against only one type of virus. Therefore, the fact is that, for the current situation where plural types of viruses are spreading at the same time, the effectiveness thereof is limited.

In addition, an anti-influenza agent capable of responding to a variation in the host range due to influenza virus transmission among animal species or a variation in antigenicity due to an influenza virus antibody that a host possesses has not been developed yet.

Therefore, the invention of this application makes it an object to solve the problems as above, and to provide a substance which can respond to a variation in the host range of an influenza virus or a variation in antigenicity and is useful as a drug or an adsorbent in a virus removal filter or the like capable of preventing infection with a type A influenza virus and a type B influenza virus originating in any animals including humans and thus preventing a flu epidemic.

### Disclosure of Invention

To solve the foregoing problems, the invention of this application firstly provides a novel branched sialo-sugar molecule characterized by being represented by the following formula (I): 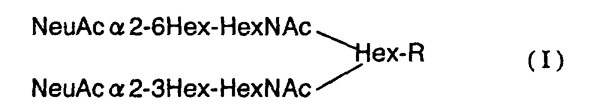 (wherein NeuAc represents *N*-acetylneuraminic acid in which the hydroxyl group, the carboxyl group and the amido group may be chemically modified with a halogen group, an alkyl group or an acyl group, either the same group or separate groups, Hex represents hexose, HexNAc represents *N*-acetylhexosamine and R represents a substrate selected from among a hydrogen atom, a hydrocarbon chain, a sugar chain, a lipid, a protein and a synthetic polymer, and R may have a substituent).

The invention of this application provides secondly the foregoing novel branched sialo-sugar molecule, in which the *N*-acetylneuraminic acid and hexose are linked by a natural O-glycoside linkage, thirdly the foregoing novel branched sialo-sugar molecule, in which the linkage between *N*-acetylneuraminic acid and hexose is a chemically converted linkage, and fourthly the foregoing novel branched sialo-sugar molecule, in which the linkage form between *N*-acetylneuraminic acid and hexose is an S-glycoside linkage or a Se-glycoside linkage.

The invention of this application provides fifthly a novel branched sialo-sugar molecule characterized by being represented by the following formula (II): 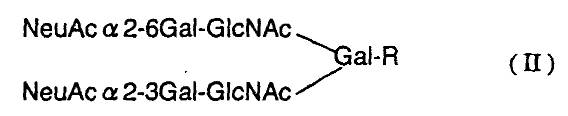 (wherein NeuAc represents *N*-acetylneuraminic acid in which the hydroxyl group, the carboxyl group and the amido group may be chemically modified with a halogen group, an alkyl group or an acyl group, either the same group or separate groups, Gal represents galactose, GlcNAc represents *N*-acetylglucosamine and R represents a substrate selected from among a hydrogen atom, a hydrocarbon chain, a sugar chain, a lipid, a protein and a synthetic polymer, and R may have a substituent).

Further, sixthly, the invention of this application provides a novel branched sialo-sugar molecule characterized by being represented by the following formula (III): 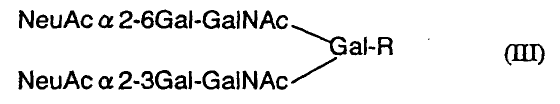 (wherein NeuAc represents *N*-acetylneuraminic acid in which the hydroxyl group, the carboxyl group and the amido group may be chemically modified with a halogen group, an alkyl group or an acyl group, either the same group or separate groups, Gal represents galactose, GalNAc represents *N*-acetylgalactosamine and R represents a substrate selected from among a hydrogen atom, a hydrocarbon chain, a sugar chain, a lipid, a protein and a synthetic polymer, and R may have a substituent).

The invention of this application provides seventhly any one of the foregoing novel branched sialo-sugar molecule, in which the *N*-acetylneuraminic acid and galactose are linked by a natural O-glycoside linkage, eighthly any one of the foregoing novel branched sialo-sugar molecule, in which the linkage between *N*-acetylneuraminic acid and galactose is a chemically converted linkage, and furthermore ninthly one of the foregoing novel branched sialo-sugar molecules, in which the linkage form between *N*-acetylneuraminic acid and galactose is an S-glycoside linkage or a Se-glycoside linkage.

And the invention of this application also provides tenthly an antiviral agent characterized by comprising at least one of the foregoing novel branched sialo-sugar molecule as an active ingredient.

### Brief Description of Drawings

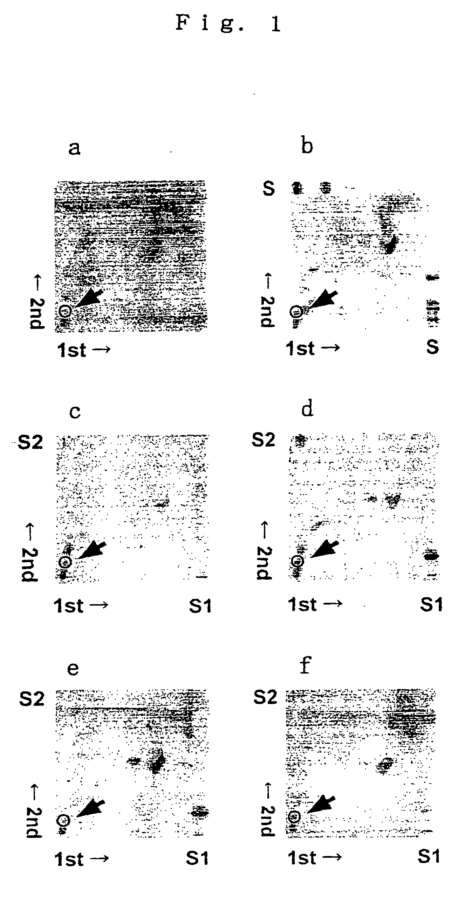

Fig. 1 shows photographs showing the results of a binding assay (two-dimensional development) of an acidic glycolipid originating in chorioallantoic membrane of embryonated hen eggs to type A influenza viruses and type B influenza viruses in an Example of the invention of this application (a: virus (-), b: color development with orcinol-H₂SO₄ reagent c: binding ability to A/PR/8/34, d: binding ability to A/Aichi/2/68, e: binding ability to B/Lee/40 f: binding ability to B/Gifu/2/73, S: standard glycolipid = bovine brain total gangliosides, S1: α 2-6 sialylparagloboside, S2: α 2-3 sialylparagloboside, 1st: one-dimensional development (CHCl₃/ MeOH/ 88% HCOOH (65/25/10, v/v/v)), 2nd : two-dimensional development (CHCl₃/ MeOH/ 5 N NH₄OH (5/4/1, v/v/v)); arrow: glycolipid that reacted with the influenza viruses in the same way).

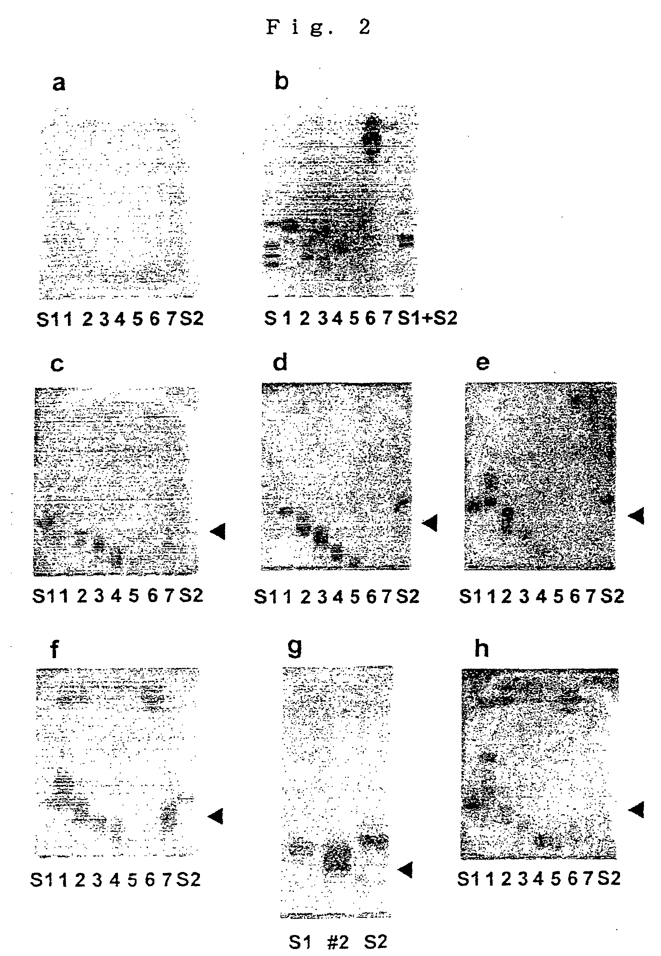

Fig. 2 shows photographs showing the results of a binding assay (CHCl₃/MeOH/ 12 mM MgCl₂ (5/4/1, v/v/v)) of an acidic glycolipid originating in chorioallantoic membrane of embryonated hen eggs purified by Iatrobeads column chromatography to type A influenza viruses and type B influenza viruses in Example of the invention of this application (a: virus (-), b: color development with orcinol-H₂SO₄ reagent, c: binding ability to A/Memphis/1/71, d: binding ability to A/PR/8/34, e: binding ability to A/Aichi/2/68, f: binding ability to A/Duck/313/4, g: binding ability to A/Duck/92/1/76, h: binding ability to B/Lee/40, S: standard glycolipid = bovine brain total gangliosides + GM3, S1: α 2-6 sialylparagloboside, S2: α 2-3 sialylparagloboside; 1 to 7: fraction; arrow: target glycolipid).

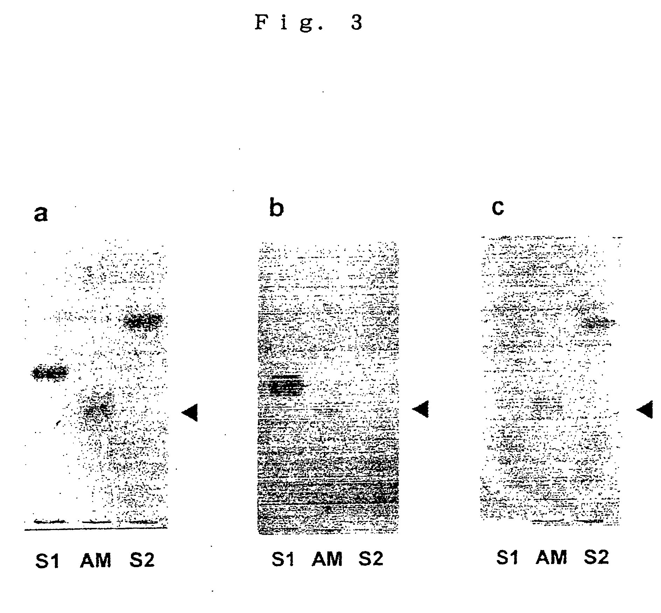

Fig. 3 shows photographs showing the results of a binding assay (CHCl₃/MeOH/ 12 mM MgCl₂/ 15 M NH₄OH (50/40/7/3, v/v/v)) of an acidic glycolipid originating in chorioallantoic membrane of embryonated hen eggs to type A influenza viruses in an Example of the invention of this application (a: color development with orcinol-H₂SO₄ reagent, b: binding ability to A/Memphis/1/71, c: binding ability to A/PR/8/34, S1: α 2-6 sialylparagloboside, S2: α 2-3 sialylparagloboside; AM: purified glycolipid originating in chorioallantoic membrane of embryonated hen eggs).

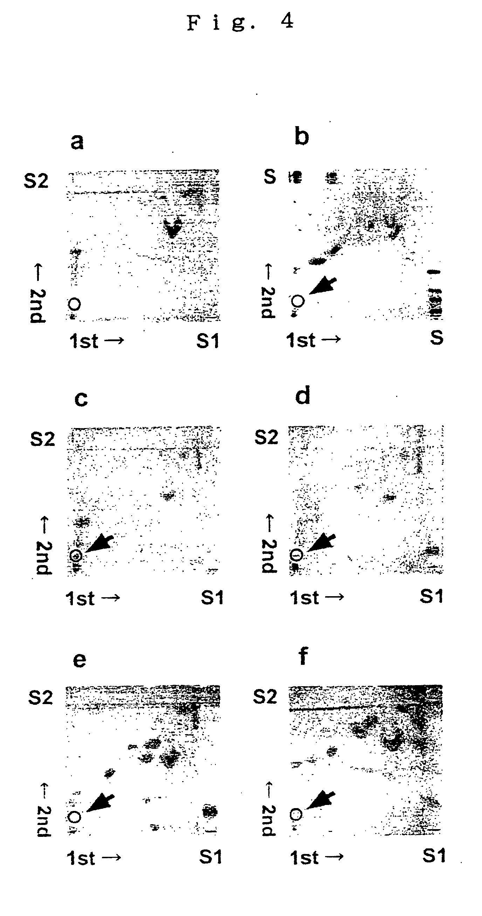

Fig. 4 shows photographs showing the results of a binding assay (two-dimensional development) of an acidic glycolipid originating in MDCK cells to type A influenza viruses and type B influenza viruses in an Example of the invention of this application (a: virus (-), b: color development with orcinol-H₂SO₄ reagent c: binding ability to A/PR/8/34, d: binding ability to A/Aichi/2/68, e: binding ability to B/Lee/40 f: binding ability to B/Gifu/2/73, S: standard glycolipid = bovine brain total gangliosides, S1: α 2-6 sialylparagloboside, S2: α 2-3 sialylparagloboside, 1st: one-dimensional development (CHCl₃/ MeOH/ 88% HCOOH (65/25/10, v/v/v)), 2nd : two-dimensional development (CHCl₃/ MeOH/ 5 N NH₄OH (5/4/1, v/v/v)); arrow: glycolipid that reacted with the influenza viruses in the same way).

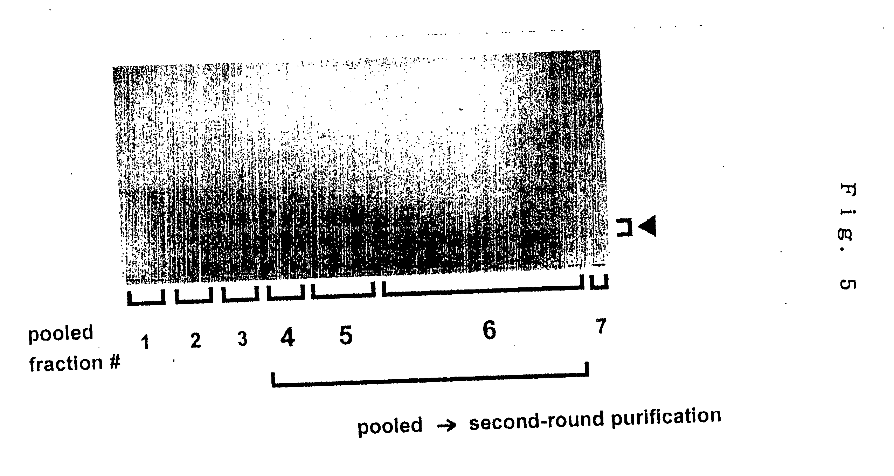

Fig. 5 is a photograph showing the elution profile of Iatrobeads column chromatography of an acidic glycolipid originating in chorioallantoic membrane of embryonated hen eggs in Example of the invention of this application (developing solvent: CHCl₃/ MeOH/ 12 mM MgCl₂ (5/4/1, v/v/v); color development with orcinol-H₂SO₄ reagent), (arrow: glycolipid showing reactivity with the influenza viruses; 1 to 7: fraction).

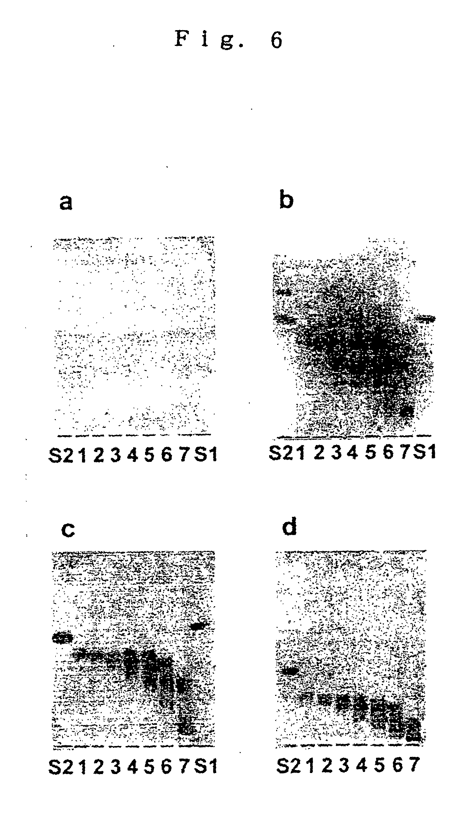

Fig. 6 shows photographs showing the results of a binding assay (CHCl₃/MeOH/ 12 mM MgCl₂ (5/4/1, v/v/v)) of an acidic glycolipid originating in chorioallantoic membrane of embryonated hen eggs purified by Iatrobeads column chromatography to type A influenza viruses in an Example of the invention of this application (a: virus (-), b: color development with orcinol-H₂SO₄ reagent, c: binding ability to A/Aichi/2/68, d: binding ability to A/Memphis/1/71, S1: α 2-6 sialylparagloboside, S2: α 2-3 sialylparagloboside; 1 to 7: various fractions).

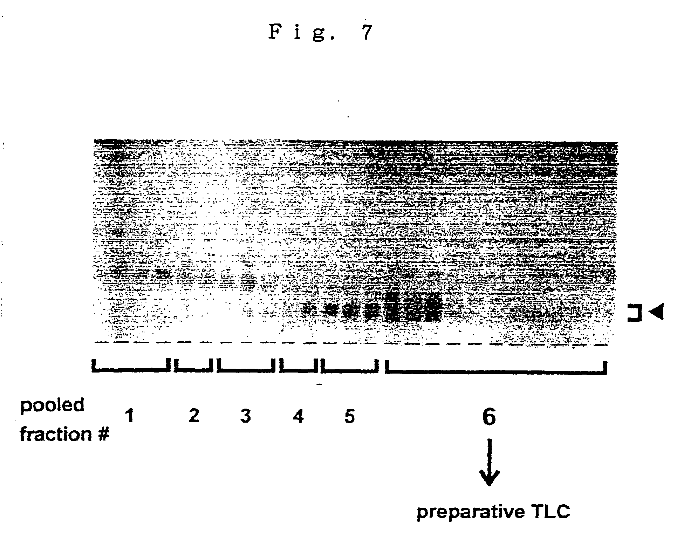

Fig. 7 is a photograph showing the elution profile of Iatrobeads column chromatography of a pooled glycolipid in an Example of the invention of this application (developing solvent: CHCl₃/ MeOH/ 12 mM MgCl₂ (5/4/1, v/v/v); color development with orcinol-H₂SO₄ reagent), (arrow: glycolipid showing reactivity with the influenza viruses).

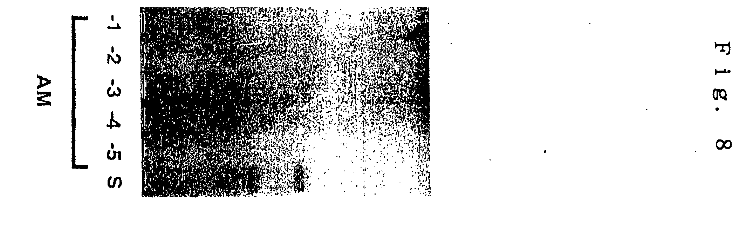

Fig. 8 is a photograph showing the TLC results of a glycolipid purified by TLC for preparation in Example of the invention of this application (developing solvent: CHCl₃/ MeOH/ 12 mM MgCl₂ (5/4/1, v/v/v); color development with orcinol-H₂SO₄ reagent), (S: bovine brain total gangliosides).

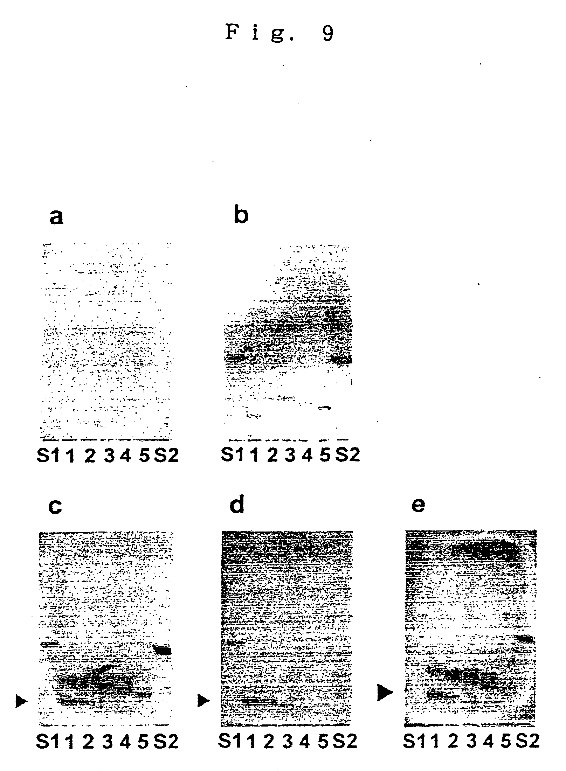

Fig. 9 shows photographs showing the results of a binding assay of a glycolipid originating in chorioallantoic membrane of embryonated hen eggs purified by TLC for preparation to type A influenza viruses in an Example of the invention of this application (a: virus (-), b: color development with orcinol-H₂SO₄ reagent, c: binding ability to A/Aichi/2/68, d: binding ability to A/PR/8/34, e: binding ability to A/Memphis/1/71, S1: α 2-6 sialylparagloboside, S2: α 2-3 sialylparagloboside; 1 to 5: AM number; arrow: glycolipid that reacted specifically with all influenza viruses).

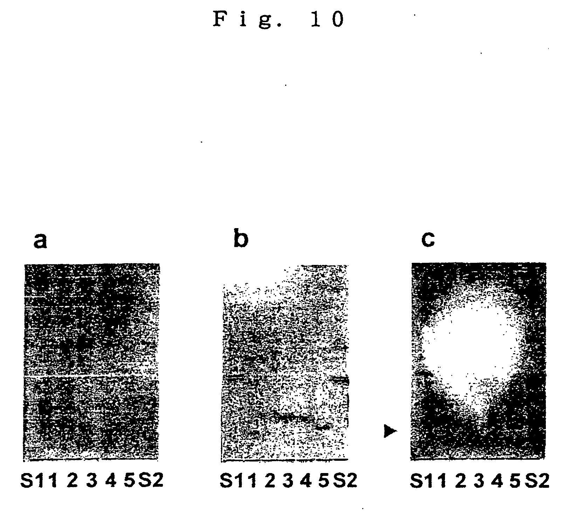

Fig. 10 shows photographs showing the results of a binding assay of a glycolipid originating in chorioallantoic membrane of embryonated hen eggs purified by TLC for preparation to B/Gifu/2/73 in an Example of the invention of this application (a: virus (-), b: color development with orcinol-H₂SO₄ reagent, c: binding ability to B/Gifu/2/73, S1: α 2-6 sialylparagloboside, S2: α 2-3 sialylparagloboside; 1 to 5: AM number; arrow: glycolipid that reacted specifically with B/Gifu/2/73).

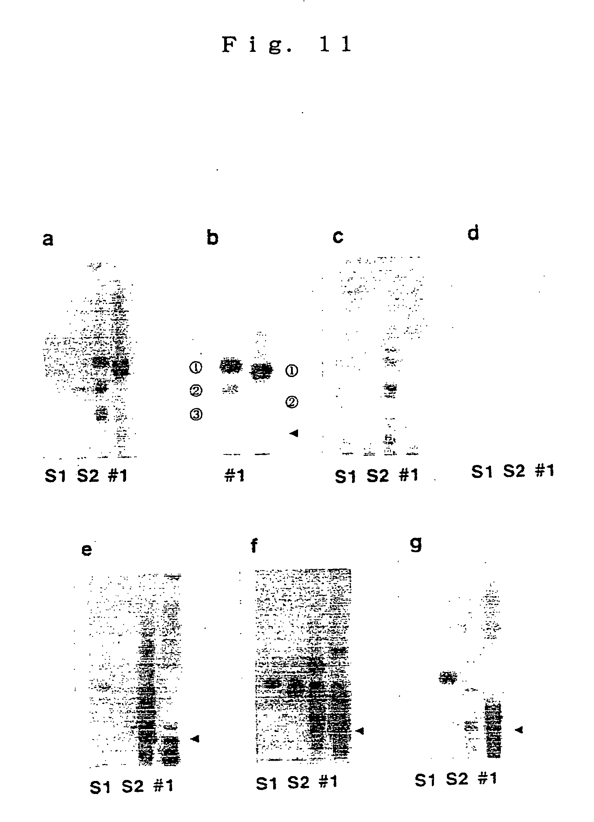

Fig. 11 shows photographs showing the results of a binding assay of a glycolipid (monosialo and disialo fractions) originating in chorioallantoic membrane of embryonated hen eggs prepared with a DEAE-Sephadex A-25 column to each influenza virus in Example of the invention of this application (a: color development with orcinol-H₂SO₄ reagent, b: color development with resorcinol-HCl reagent, c: virus (-) (primary antibody = anti-P-50), d: virus (-) (primary antibody = USA1), e: binding ability to A/PR/8/34 (H1N1), f: binding ability to A/Aichi/2/68 (H3N2), g: binding ability to A/Memphis/1/71 (H3N2); S1: GSC273 (α 2-3 sialylparagloboside), S2: GSC275 (α 2-6 sialylparagloboside); #1: monosialo fraction; #2: disialo fraction).

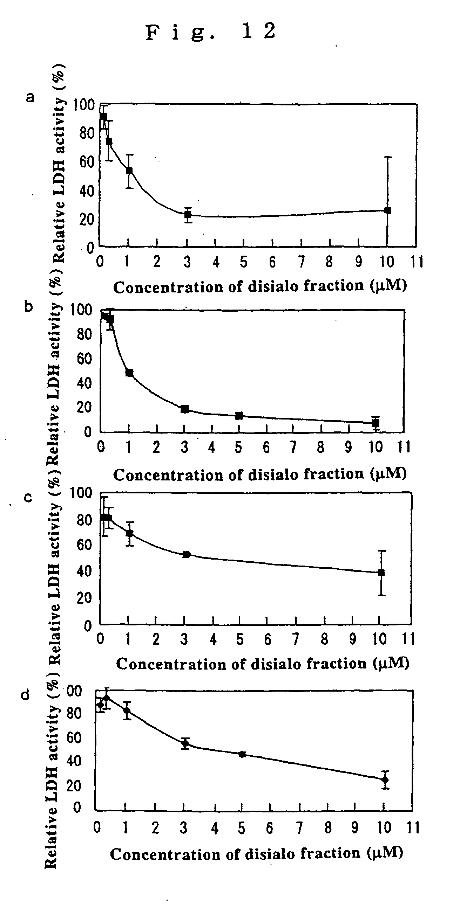

Fig. 12 shows graphs showing the inhibitory activity of a disialo fraction of chorioallantoic membrane on infection with various influenza viruses (relative activity when a disialo fraction of each concentration was added, where the LDH activity when only a virus solution was added is 100%). (a: binding ability to A/PR/8/34, b: binding ability to A/Aichi/2/68, c: binding ability to A/Memphis/1/71, d: binding ability to B/Lee/40)

### Best Mode for Carrying Out the Invention

The inventors of this application has implemented an analysis of influenza virus genes and an investigation on sialo-sugar chains that become the virus receptor required for infection of a host, and revealed the mechanism of transmitting an influenza virus from humans to other animals (Ito, T., Suzuki, Y., Takada, A., Kawamoto, A., Otsuki, K., Masuda, H., Yamada, M., Suzuki, T., Kida, H., and Kawaoka, Y.: *Journal of Virology,* 71, 3357-3362 (1997); Suzuki, T., Horiike, G., Yamazaki, Y., Kawabe, K., Masuda, H., Miyamoto, M., Nishimura, S.I., Yamagata, T., Ito, T., Kida, H., Kawaoka, Y., and Suzuki, Y.: *FEBS Letters*, 404, 192-196 (1997); Ito, T., Suzuki, Y., Mitnaul, L., Vines, A., Kida, H., and Kawaoka, Y.: *Virology,* 227, 493-499 (1997): Suzuki, Y.: Options for the control of influenza III. (Elsevier Science B.V., Brown, L.E., Hampson, A.W., Webster, R.G., editors), 4443-4446 (1996)). They revealed that all of the type A influenza viruses isolated from humans, pigs, birds and horses, and type B human influenza viruses specifically recognize and bind to a sugar chain containing a sialic acid (SA) in common, in particular, strongly bind to sialyl lacto-series type I and type II sugar chains (type I sugar chain: SA α 2-6(3)Gal β 1-3GlcNAc β 1-; type II sugar chain: SA α 2-6(3)Gal β 1-4GlcNAc β 1-) (Suzuki, Y., Nakao, T., Ito, T., Watanabe, N., Toda, Y., Xu, G., Suzuki, T., Kobayashi, T., Kimura, Y., Yamada, A., et al.: *Virology,* 189, 121-131 (1992): Suzuki Yasuo: Seikagaku, 62, 231-260 (1990)).

Further, the inventors found that in the case where an active sialo-sugar chain is present as a free oligosaccharide, its binding ability to an influenza virus is weak, however, in the case where it is present as a sugar chain bound to a lipid or a protein such as a sialoglycolipid or a sialoglycoprotein, and further in the case where the foregoing sialo-sugar chains are expressed to form a cluster on a chemically synthesized polymer, it shows a remarkably high binding ability (Fujimoto, K., Hayashida, O., Aoyama, Y., Guo, C.T., Hidari, K.I.P.J., and Suzuki, Y.: *Chemistry Letter,* 1259-1260 (1999); Akiko Tsuchida, Kazukiyo Kobayashi, Noritaka Matsubara, Tsukasa Muramatsu, Takashi Suzuki, Yasuo Suzuki: *Glycoconjugate J.,* 15, 1047-1054 (1998)).

Furthermore, they also found that the molecular evolution of hemagglutinin of type A and type B influenza viruses expresses a change in recognition of the sialic acid linkage type of the terminal of receptor sialo-sugar chains (SA2-3Gal, SA2-6Gal) and the sialic acid molecular species (Neu5Ac, Neu5Gc) (Suzuki, Y., Kato, H., Naeve, C.W., Webster, R.G.: *Virology*, 63, 4298-4302 (1989): Xu, G., Horiike, G., Suzuki, T., Miyamoto, D., Kumihashi, H., and Suzuki, Y.: *Biochemical and biophysical research communications*, 224, 815-818 (1996)). For example, type A (H1 and H3 subtype) and type B viruses isolated from humans with the use of MDCK cells recognize SA2-6Gal linkage (hereinafter referred to as 2-6); on the other hand, all the viruses isolated from ducks and horses show a strong affinity to SA2-3Gal linkage (hereinafter referred to as 2-3), and their binding ability to 2-6 is weak. Meanwhile, viruses isolated from pigs show a strong affinity to 2-6, however, they can also bind to 2-3.

Therefore, they investigated the linkage type and molecular species of sialic acid in the structure of an influenza virus receptor (cells of upper respiratory mucosa for humans, pigs and horses, cells of the intestinal mucosa for ducks), and revealed that receptor sialo-sugar chains (both 2,6- and 2,3-) for both human and duck viruses are present in cells of upper respiratory tract of pigs. In other words, it was reveled that pigs can become an intermediate host of naturally occurring duck and human influenza viruses at the molecular level.

However, in fact, the existence or the structure of a sialo-sugar chain (namely, a specific receptor molecule) expressed on the surface of a host cell has not been revealed so far. Therefore, the inventors of this application, by intensive studies, extracted a variety of sialo-sugar chains from tissues infected with influenza viruses and investigated the binding ability to the respective influenza viruses, thereby achieving the invention of this application.

Namely, a novel branched sialo-sugar molecule of the invention of this application is the one represented by the following chemical formula (I): 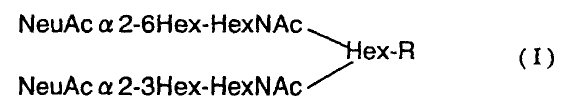 (wherein NeuAc represents *N*-acetylneuraminic acid in which the hydroxyl group, the carboxyl group and the amido group may be chemically modified with a halogen group, an alkyl group or an acyl group, either the same group or separate groups, Hex represents hexose, HexNAc represents *N*-acetylhexosamine and R represents a substrate selected from among a hydrogen atom, a hydrocarbon chain, a sugar chain, a lipid, a protein and a synthetic polymer, and R may have a substituent). The novel branched sialo-sugar molecule of the invention of this application is specifically characterized by having both NeuAc linked to Hex-HexNAc through an α 2,6 linkage and NeuAc linked to Hex-HexNAc through an α 2,3 linkage.

In such a compound, the type of Hex and HexNAc is not particularly limited. Preferably, Hex is galactose (Gal), HexNAc is *N*-acetylgalactosamine (GalNAc) or *N*-acetylglucosamine (GluNAc). In addition, NeuAc may be the one in which the hydroxyl group, the carboxyl group and the amido group may be chemically modified with a halogen group (F, Cl, Br or I), an alkyl group (C₂ to C₂₀) or an acyl group (C₂ to C₂₀).

Further, in the novel branched sialo-sugar molecule of the invention of this application, the linkage between *N*-acetylneuraminic acid and hexose (or galactose) may not only be an O-glycoside linkage occurring in nature but also be chemically converted into an S-glycoside, a Se-glycoside linkage or the like.

In addition, in the novel branched sialo-sugar molecule of the invention of this application, R may be a hydrogen atom or a free oligosaccharide which is a hydrocarbon group which may have a substituent, or the derivative thereof, or R may be a sugar chain, a lipid, a protein, a synthetic polymer or the like. In the case R having a lipid, a protein, a synthetic polymer or the like as described above, and forming a novel branched sialoglycolipid, a novel branched sialoglycoprotein, a novel branched sialo sugar for immobilizing a polymer or the like, the binding ability to an influenza virus becomes high, therefore, it is preferable. Also, the structure of the R may be appropriately changed according to the usage of the novel branched sialo-sugar molecule of this invention.

The novel branched sialo-sugar molecule of the invention of this application may be the one which is synthesized by a known synthetic method of a polysaccharide, or the one which is derived from nature. In particular, the one obtained by isolating one originating in chorioallantoic membrane of an embryonated hen egg is exemplified.

The one novel branched sialo-sugar molecule of the invention of this application has the plural sialo-sugar chains, in particular those with α 2,6- and α 2,3- linkages, which are necessary for all the types (type A, type B and hemagglutinin subtype of type A influenza viruses) of viruses to bind to a host cell receptor, whereby it binds effectively to any of the type A influenza viruses and type B influenza viruses.

Therefore, the novel branched sialo-sugar molecule of the invention of this application can be applied to various means for preventing the infection with an influenza virus and so preventing a flu epidemic. Its applications include an active ingredient of a drug (antiviral agent) and a virus adsorbent in a filter such as a face guard or an air purification system. For example, in the case where it is used as an antiviral agent, it may be any form, including the one which is orally administered such as an liquid, a tablet or a powder, the one which is directly applied to the nasal cavity or the throat such as an aerosol including a nasal drop, an oral spray and the like, the one which is transdermally administered, and the like. In addition, in the case where it is filled in a filter or the like, it is possible to remove viruses from air and to prevent the diffusion of the virus. Further, if the novel branched sialo-sugar molecule of the invention of this application is mixed in or applied on a household appliance such as wallpaper, it is possible to give it the effect of removing viruses and preventing their diffusion.

The novel branched sialo-sugar molecule of this invention has a binding ability to an influenza virus equal to or greater than a conventional linear sugar chain group, and exerts high efficacy in preventing infection. In addition, as described above, it has a high binding ability to any influenza viruses, therefore, even if plural types of flu epidemics occur simultaneously, or a new variant of an influenza virus is generated, a sufficient binding ability can be maintained, and its function as an antiviral agent or a virus adsorbent can be achieved; therefore, its usefulness is higher than a conventional linear sugar chain group.

Hereunder, the invention of this application will be explained in more detail by showing Examples. Of course, it is without saying that the invention of this application is not limited to the following Examples.

### Examples

### Example 1 Search for candidate molecule for influenza virus receptor

To search and identify a molecule to become a receptor for an influenza virus, the attempt was made to isolate lipid molecules that react with type A and type B influenza viruses from chorioallantoic membrane of embryonated hen eggs used for virus growth and Madin Darby canine kidney cells (MDCK cells) used for virus isolation or infection experiments.

### I. Experimental method

### (1) Materials

As an influenza virus strain, the ones shown in Table 1 were used.

**Table 1**

| Virus type | Serotype | Binding specificity to NeuAc |
|---|---|---|
| A/PR/8/34 | H1N1 | α 2-6 << α 2-3 |
| A/Aichi/2/68 | H3N2 | α 2-6 > α 2-3 |
| A/Memphis/1/71 | H3N2 | α 2-6 >> α 2-3 |
| A/Duck/313/4 | H5N3 | α 2-6 ≈ α 2-3 |
| A/Duck/92/1/76 | H9N2 | α 2-6 ≈ α 2-3 |
| B/Lee/40 | | α 2-6 >> α 2-3 |
| B/Gifu/2/73 | | α 2-6 ≈ α 2-3 |

In the table, the binding property to *N*-acetylneuraminic acid is the value based on the results of examining the reactivity with a standard glycolipid (sialylparagloboside) by a TLC/virus-binding assay described later in III. The TLC/virus-binding assay is a method established by the inventors (Suzuki, Y., Nakao, T., Ito, T., Watanabe, N., Toda, Y., Xu, G. Y., Suzuki, T., Kobayashi, T., Kimura, Y., Yamada, A., Sugawara, K., Nishimura, H., Kitame, F., Nakamura, K., Deya, E., Kiso, M., and Hasegawa, A. (1992) *Virology* 189, 121-131; Guo, C.-T., Wong, C.-H., Kajimoto, T., Miura, T., Ida, Y., Juneja, L.R., Kim, M.-J., Masuda, H., Suzuki, T., and Suzuki, Y. (1998) *Glycoconjugate* J. 15, 1099-1108).

As an anti-influenza virus antibody, the ones shown in the following Table 2 were used.

**Table 2**

| Virus type | Primary antibody*¹ | Secondary antibody*² |
|---|---|---|
| A/PR/8/34 | anti-P-50 (x 1000) | Protein A (x 1000) |
| A/Aichi/2/68 | anti-P-50 (x 1000) | Protein A (x 1000) |
| A/Memphis/1/71 | USA 1 (x 500) | Protein A (x 1000) |
| A/Duck/313/4 | anti-P-50 (x 1000) | Protein A (x 1000) |
| A/Duck/92/1/76 | anti-P-50 (x 1000) | Protein A (x 1000) |
| B/Lee/40 | anti-B/Lee (x 4000) | anti-rabbit IgG-HRP (x 2000) |
| B/Gifu/2/73 | anti-B/Lee (x 4000) | anti-rabbit IgG-HRP (x 2000) |

| | | |
|---|---|---|
| *1: The primary antibody is a rabbit polyclonal antibody produced by the inventors. | | |
| *2: anti-rabbit IgG-HRP (purchased from Santa Cruz Biotechnology), Protein A (purchased from Cappel) | | |

### (2) Purification of influenza virus

A 0.2 ml portion of virus suspension diluted with PBS (type A influenza virus: 2-3 to 4; type B influenza virus: 2-1 to 0) was inoculated into the chorioallantoic cavity of 11-day old embryonated hen eggs and incubated at 34°C for 48 hours.

After chorioallantoic fluid was recovered, the fluid was centrifuged at 4°C for 10 minutes at 4000 rpm (HITACHI SCI 20B, RPR9 rotor) to remove impurities. The supernatant was further centrifuged at 4°C for 3 hours at 8000 rpm, and the pellet was suspended in PBS and used as a crude virus solution. Subsequently, the crude virus was deposited in a layer on 50 % glycerol (WAKO) in PBS, and centrifuged at 4°C for 2 hours at 38000 rpm (HITACHI CP65β, P40ST rotor). The obtained pellet was suspended in PBS again, and 10-40% (w/v) continuous sucrose density gradient centrifugation was carried out at 4°C for 90 minutes at 20000 rpm (HITACHI CP65β, P28S rotor).

The band of virus was collected, and by performing centrifugation at 4°C for 3 hours at 8000 rpm (HITACHI SCR 20B, RPR18 rotor), a purified virus was obtained. The obtained virus was suspended in PBS and stored at -80°C.

### (3) Measurement of hemagglutination activity (HAU)

A 50 µl portion of the purified influenza virus diluted 210-fold was serially diluted 2-fold with 50 µl of 0.01 % gelatin (nacalai tesque, Inc.) in PBS on a microplate (96 U-bottom wells, Falcon). A 50 µl portion of 0.5% (v/v) guinea pig erythrocytes in PBS was added, left to stand at 4°C for 2 hours, and then hemagglutination was monitored. HAU was expressed as the maximum dilution of the virus required for causing agglutination of guinea pig erythrocytes.

### (4) Search for reactive lipid from chorioallantoic membrane

Chorioallantoic membrane was collected from 40 embryonated hen eggs of 11-day old, washed with PBS, and freeze-dried (EYELA FDU-830). To 1.81 g (dry weight) of chorioallantoic membrane, 15 ml of MilliQ water was added and homogenized. Further, 60 ml of CHCl₃/MeOH was added, and extracted for 1 hour. After the extract was filtered, the solvent was removed with an evaporator. Subsequently, 60 ml of MeOH was added, and sonication was carried out by using a bath sonicator (SILENTSONIC, SHARP), then hydrolysis was carried out overnight with 0.1 N NaOH at 37°C. After the solvent was removed, 20 ml of MilliQ water was added, sonicated, and dialysis was carried out (dialysis membrane: VISKASE SALES CORP).

After the dialysis, the freeze-dried sample was dissolved in 20 ml of CHCl₃/ MeOH/ MilliQ water (30/60/8, v/v/v), sonicated, and used as the sample for DEAE- Sephadex A-25 column chromatography. DEAE-Sephadex A-25 (Pharmacia Biotech) was used after it was activated with CHCl₃/ MeOH/ 0.8 M NaOAc (30/60/8, v/v/v) and equilibrated with CHCl₃/ MeOH/ MilliQ water (30/60/8, v/v/v). The sample was applied to DEAE-Sephadex A-25 with 10 ml of bed volume, and a neutral lipid fraction was eluted with 100 ml of CHCl₃/MeOH/ MilliQ water (30/60/8, v/v/v), and then an acidic lipid fraction was eluted with 100 ml of CHCl₃/ MeOH/ 0.8 M NaOAc (30/60/8, v/v/v). The solvent was removed from these fractions, and 10 ml of MilliQ water was added, sonicated, then dialysis was carried out.

After the dialysis, furthermore, half of the acidic lipid fraction among the freeze-dried samples was dissolved in 0.75 ml of CHCl₃/ MeOH/ MilliQ water (5/4/1, v/v/v), and applied to Iatrobeads (3 ml of bed volume) (Iatron Laboratories, Inc.), which had been equilibrated with CHCl₃/ MeOH/ MilliQ water (5/4/1, v/v/v) beforehand.

First, 1 ml of each fraction was collected while 15 ml of CHCl₃/ MeOH/MilliQ water (5/4/1, v/v/v) was passed through. Thereafter, 15 ml of CHCl₃/MeOH/ MilliQ water (30/60/8, v/v/v) and 15 ml of MeOH were passed through. Among these fractions, fractions containing the target glycolipid were collected, dissolved in 1 ml of CHCl₃/ MeOH (1/1, v/v), and an analysis was carried out by two-dimensional development on a TLC plate, which will be described later in II (a) and III.

To Iatrobeads (25 ml bed volume) equilibrated with CHCl₃/ MeOH/MilliQ water (5/4/1, v/v/v) beforehand, 2.5 ml of a sample dissolved in CHCl₃/MeOH/ MilliQ water (5/4/1, v/v/v) was applied. Elution was carried out with gradients of solvents from 125 ml of CHCl₃/ MeOH/ MilliQ water (5/4/1, v/v/v) to 125 ml of CHCl₃/ MeOH/ MilliQ water (30/60/8, v/v/v), and the eluate was fractionated into 2.5 ml fractions by using a fraction collector (ADVANTEC, SF-3120). For these fractions, the reactivity with type A (human, bird) and type B viruses was investigated by the analytical methods described later in II (b) and III.

To further purify the fraction for which results have been confirmed, the whole amount was applied to an HPTLC (MERCK, HPTLC-Fertigplatten Kieselgel 60) plate and developed with CHCl₃/ MeOH/ 12 mM MgCl₂/ 15 M NH₄OH (50/40/7/3, v/v/v/v), and the obtained four bands were scraped off and collected by using a surgical knife (KEISEI MEDICAL INDUSTRIAL CO., LTD). To the respective collected bands, 1 ml of CHCl₃/ MeOH/ MilliQ water (5/4/1, v/v/v) was added, sonicated for 5 minutes, and filtered through a cotton plug by using a Pasteur pipette. Further, 2 ml of CHCl₃/ MeOH/ MilliQ water (30/60/8, v/v/v) was passed through a cotton plug and combined with the previous filtrate. After the solvent was removed, 100 µl of CHCl₃/ MeOH (1/1, v/v) was added, and then an analysis was carried out on a TLC plate by the methods described later in II (c) and III.

### (5) Research for reactive lipid from MDCK cells

MDCK cells were cultured in a 10 cm dish (total of 25 dishes) until confluent, and collected. The obtained cell pellet was freeze-dried (dry weight of 0.06 g), and 600 µl of MilliQ water was added. Further, 6 ml of CHCl₃/MeOH (1/1, v/v) was added, centrifuged at 1500 rpm (TOMY, LC-100) for 5 minutes, and the solvent of the obtained supernatant was removed. Then, 3 ml of MeOH was added, sonicated, and hydrolysis was carried out overnight with 0.1 N NaOH at 37°C. After the solvent was removed, 2.5 ml of MilliQ water was added, sonicated, and dialysis was carried out.

After the dialysis, the freeze-dried sample was dissolved in 20 ml of CHCl₃/ MeOH/ MilliQ water (30/60/8, v/v/v), sonicated, and used as the sample for DEAE-Sephadex A-25 column chromatography. DEAE- Sephadex A-25 was used after it was activated with CHCl₃/ MeOH/ 0.8 M NaOAc (30/60/8, v/v/v) and this was replaced with CHCl₃/ MeOH/ MilliQ water (30/60/8, v/v/v). The sample was applied to DEAE-Sephadex A-25 with 10 ml bed volume, and a neutral lipid fraction was eluted with 100 ml of CHCl₃/ MeOH/ MilliQ water (30/60/8, v/v/v).

Thereafter, an acidic lipid fraction was eluted with 100 ml of CHCl₃/MeOH/ 0.8 M NaOAc (30/60/8, v/v/v). The solvent was removed from these fractions, and 2.5 ml of MilliQ water was added, sonicated, then dialysis was carried out. After the dialysis, the freeze-dried acidic lipid fraction was dissolved in 100 µl of CHCl₃/ MeOH (1/1, v/v), and an analysis was carried out by the two-dimensional development on a TLC plate described in II (a) and III.

### II. Detection with chemical coloring reagent for TLC

(a) Two-dimensional development: After a plastic TLC plate (MERCHEREY-NAGEL, Polygram Sil G) was developed with acetone, the obtained acidic lipid fraction and the standard glycolipid (bovine brain total gangliosides) were applied thereto. After this TLC plate was developed one-dimensionally with CHCl₃/ MeOH/ 88% HCOOH (65/25/10, v/v/v), it was air-dried well, then two-dimensional development was carried out with CHCl₃/MeOH/ 5 N NH₄OH. After the plate was air-dried, an orcinol-H₂SO₄ reagent (1% (w/v) orcinol/2N H₂SO₄) was sprayed, heated to 110°C, and detection of sugar was carried out.
   With regard to the fraction in which reactivity with a virus had been observed from the result of two-dimensional development, Iatrobeads column chromatography was carried out again for the purpose of further purification, after combining the fraction obtained by the foregoing Iatrobeads column chromatography with the rest of the half amount of the acidic lipid fraction.
(b) One-dimensional development: The obtained acidic lipid fraction and the standard glycolipid (GSC-275, GSC-273, bovine brain total gangliosides) were applied on a plastic TLC plate. After this TLC plate was developed with acetone, it was developed with CHCl₃/ MeOH/ 12 mM MgCl₂ (5/4/1, v/v/v). After the plate was air-dried, an orcinol-H₂SO₄ reagent was sprayed, heated to 110°C, and detection of sugar was carried out.
   Incidentally, GSC-275 is the synthetic compound, α 2,6 sialylparagloboside, represented by the following [1], and GSC-273 is the synthetic compound, α 2,3 sialylparagloboside, represented by the following [2].

   Neu5Ac α 2-6Gal β 1-4GlcNAc β 1-3Gal β 1-4Glc β 1-0CH(C₁₄H₂₉)₂ [1]

   Neu5Ac α 2-3Gal β 1-4GlcNAc β 1-3Gal β 1-4Glc β 1-0CH(C₁₄H₂₉)₂ [2]
(c) One-dimensional development: The obtained glycolipid fraction and the standard glycolipid (the foregoing orcinol-H₂SO₄ reagent) were applied on a plastic TLC plate. After this TLC plate was developed with acetone, it was developed with CHCl₃/ MeOH/ 12 mM MgCl₂ / 15 M NH₄OH (50/40/7/3, v/v/v/v). After the plate was air-dried, an orcinol-H₂SO₄ reagent was sprayed, heated to 110°C, and detection of sugar was carried out.

### III. TLC/virus-binding assay

A TLC plate developed in the same manner as in II (a) to (c) was shaken in a PBS solution of 1% ovalbumin and 1% polyvinylpyrrolidone (PVP) (Tokyo Kasei Kogyo Co., Ltd) (hereinafter referred to as A solution) at room temperature for 1 hour. After it was washed three times with PBS at 4°C for 5 minutes, 3 ml of an influenza virus suspension, which had been diluted with PBS so as to become HAU = 27, was added, and shaken at 4°C for one night. Incidentally, to the virus (-), PBS was added instead of a virus suspension.

The plate was washed three times with PBS at 4°C for 5 minutes, and 3 ml of a primary antibody solution diluted with 3% PVP-PBS solution (hereinafter referred to as B solution) was added, then shaken at 4°C for 2 hours. The plate was washed three times with PBS at 4°C for 5 minutes, and 3 ml of a secondary antibody solution diluted with B solution was added, then shaken at 4°C for 2 hours. Thereafter, the plate was washed three times with PBS at 4°C for 5 minutes, a coloring substrate solution (0.1 M citratate buffer pH 6.0, 5 ml; 0.11 mM 4-chloro-1- naphthol in CH₃CN, 100 µl; 0.06 M DEPDA in CH₃CN, 100 µl; 100 µl; 30% H₂O₂, 5 µl) was added and shaken at room temperature. After color appeared, the plate was washed with purified water and air-dried.

### IV. Results

Lipid was extracted from chorioallantoic membrane of embryonated hen eggs, and an acidic lipid fraction was two-dimensionally developed by TLC, thereafter, the color was developed (Fig. 1c) with an orcinol-H₂SO₄ reagent, which is a reagent for detecting sugar. When a plate developed in the same manner was reacted with A/PR/8/34 (Fig. 1d), A/Aichi/2/68 (Fig. 1e), B/Lee/40 (Fig. 1f), and B/Gifu/2/73 (Fig. 1g), spots showing reaction with viruses in the same way (indicated with the arrows in the figure) were confirmed.

Judging from the mobility, the fraction that reacted in all cases is a molecule different from sialylparagloboside. It was indicated that it is a substance having a longer sugar chain structure.

Therefore, in order to further purify this fraction, Iatrobeads column chromatography was carried out. The obtained patterns were shown in Fig. 2a.

With regard to the fractions 1 to 7, reactivity to type A was investigated with the use of, A/Memphis/1/71 (Fig. 2c), A/PR/8/34 (Fig. 2d), A/Aichi/2/68 (Fig. 2e), A/Duck/313/4 (Fig. 2f), A/Duck/92/1/76 (Fig. 2g), and for type B, B/Lee/40 (Fig. 2h) was used.

A/Memphis/1/71 (Fig. 2c) that strongly reacts with the sugar chain structure of Neu5Ac α 2-6Gal linkage reacted with fractions 2 to 6, the band indicated by the arrow in fraction 2 being very sharp. A/Aichi/2/68 (Fig. 2e) that reacts with the sugar chain structure of Neu5Ac α 2-6Gal linkage to the same degree also strongly reacted with the band indicated by the arrow in fraction 2 in the same manner as A/Memphis/1/71. This band also reacted with A/Duck/313/4 (Fig. 2f), A/Duck/92/1/76 (Fig. 2g), B/Lee/40 (Fig. 2h), and it was demonstrated that this band not only reacts with human type A and type B influenza viruses in the same way, but also reacts with bird viruses, H5 and H9.

Therefore, in order to further purify this reactive lipid, fraction 2 was scraped off and collected in HPTLC, and the pattern shown in Fig 3a was obtained. It was confirmed that this band reacts with A/Memphis/1/71 (Fig. 3b) and A/PR/8/34 (Fig. 3c).

On the other hand, lipid was extracted from MDCK cells in the same manner, an acidic lipid fraction was two-dimensionally developed by TLC, and the color was developed with an orcinol-H₂SO₄ reagent, thereby obtaining the pattern as shown in Fig. 4a. A plate that had been developed in the same manner was reacted with A/PR/8/34 (Fig. 4c), A/Aichi/2/68 (Fig. 4d), B/Lee/40 (Fig. 4e) and B/Gifu/2/73 (Fig. 4f), it was found that the regions shown by the arrows react with all the viruses in the same way.

It was demonstrated that this reactive lipid has the same mobility as that of the reacted fraction in chorioallantoic membrane (Fig. 1).

### Example 2

From Example 1, it was confirmed that a specific lipid molecule contained in chorioallantoic membrane of embryonated hen eggs and MDCK cells reacts with type A and type B influenza viruses. To investigate the structure and the binding specificity of this lipid molecule, isolation and purification were carried out in a still larger scale. The binding specificity of the finally isolated lipid molecule to an influenza virus was compared with that of a standard glycolipid, sialylparagloboside whose binding ability to a virus has been already reported, and results were evaluated.

### I. Experimental method

### (1) Materials

As an influenza virus strain, the ones shown in Table 3 were used.

**Table 3**

| Virus type | Serotype | Binding specificity to NeuAc |
|---|---|---|
| A/PR/8/34 | H1N1 | α 2-6 << α 2-3 |
| A/Aichi/2/68 | H3N2 | α 2-6 > α 2-3 |
| A/Memphis/1/71 | H3N2 | α 2-6 >> α 2-3 |
| B/Lee/40 | | α 2-6 >> α 2-3 |
| B/Gifu/2/73 | | α 2-6 ≈ α 2-3 |

In the table, the binding property to *N*-acetylneuraminic acid is the value based on the results of examining the reactivity with a standard glycolipid, sialylparagloboside, by a TLC/virus-binding assay shown in Example 1.

As an anti-influenza virus antibody, the ones shown in the following Table 4 were used.

**Table 4**

| Virus type | Primary antibody*¹ | Secondary antibody*² |
|---|---|---|
| A/PR/8/34 | anti-P-50 (x 1000) | Protein A (x 1000) |
| A/Aichi/2/68 | anti-P-50 (x 1000) | Protein A (x 1000) |
| A/Memphis/1/71 | USA 1 (x 500) | Protein A (x 1000) |
| B/Lee/40 | anti-B/Lee (x 4000) | anti-rabbit IgG-HRP (x 2000) |
| B/Gifu/2/73 | anti-B/Lee (x 4000) | anti-rabbit IgG-HRP (x 2000) |

### (2) Purification of target lipid from chorioallantoic membrane

Chorioallantoic membrane was collected from 1150 embryonated hen eggs of 11-day old, washed with PBS, and freeze-dried. To 67.7 g (dry weight) of chorioallantoic membrane, 800 ml of acetone was added and sonicated for 10 minutes. After the mixture was homogenized by using a homogenizer, it was stirred at room temperature for 3 hours. The extract was filtered, and to the dried residue, 2750 ml of CHCl₃/ MeOH/ MilliQ water (5/5/1, v/v/v) was added, and sonicated for 10 minutes. After it was stirred for one night, 1200 ml of CHCl₃/ MeOH/ MilliQ water (30/60/8, v/v/v) was added to the filtered residue, and this was stirred for 2 hours.

In addition, the filtrate was combined with the previous filtrate, and the solvent was removed with an evaporator. Subsequently, 300 ml of MeOH was added, sonicated, and the mixture was prepared to be the final concentration of 0.1 N NaOH, then hydrolysis was carried out at 37°C for 3.5 hours, and further at room temperature overnight. After the mixture was neutralized with AcOH which had been prepared so that the final concentration would be 0.1 N, the solvent was removed. Subsequently, 80 ml of MilliQ water was added, sonicated, then dialysis was carried out. After the dialysis, the freeze-dried sample was dissolved in 100 ml of CHCl₃/ MeOH/ MilliQ water (30/60/8, v/v/v), sonicated, then filtered. To the residue, 100 ml of CHCl₃/ MeOH/ MilliQ water (30/60/8, v/v/v) was added again, and the same procedure was performed twice. The filtrates produced these 3 times were combined and used as the sample for DEAE-Sephadex A-25 column chromatography. DEAE-Sephadex A-25 was used after it was activated beforehand with CHCl₃/ MeOH/ 0.8 M NaOAc (30/60/8, v/v/v) and equilibrated with CHCl₃/ MeOH/ MilliQ water (30/60/8, v/v/v).

The sample was applied to DEAE-Sephadex A-25 with 440 ml of bed volume, and a neutral lipid fraction was eluted with 20 l of CHCl₃/ MeOH/MilliQ water (30/60/8, v/v/v). Thereafter, 9 l of CHCl₃/ MeOH/ 0.05 M NaOAc (30/60/8, v/v/v) was passed through and this was used as a monosialo lipid fraction (Suzuki, Y., Nakao, T., Ito, T., Watanabe, N., Toda, Y., Xu, G., Suzuki, T., Kobayashi, T., Kimura, Y., Yamada, A., et al.: *Virology*, 189, 121-131 (1992)).

With regard to the fractions of monosialo, di- or higher sialo (trisialo, tetrasialo,...), 150 ml of MilliQ water was added and dialysis was carried out. After the dialysis, the freeze-dried fractions of di- or higher sialo was dissolved in 10 ml of CHCl₃/ MeOH/ MilliQ water (65/25/4, v/v/v). In order to further purify the fractions of di- or higher sialo, Iatrobeads column chromatography was carried out. Iatrobeads activated beforehand with CHCl₃/ MeOH/ MilliQ water (65/25/4, v/v/v) were used. The sample was applied to Iatrobeads with 120 ml of bed volume, and 250 ml of CHCl₃/ MeOH/ MilliQ water (65/25/4, v/v/v) was passed through. At this time, the eluate was fractionated into 2.5 ml fractions and 100 fractions were collected by using a fraction collector. The eluted fractions were confirmed by using an HPTLC plate. The developing solvent for HPTLC plate was CHCl₃/ MeOH/ 12 mM MgCl₂ (5/4/1, v/v/v), and development and confirmation were carried out. In addition, the reactivity with a virus was investigated by the same method as in the foregoing Example 1, and only fractions having reactivity were collected.

These fractions were dissolved in 1 ml of CHCl₃/ MeOH/ MilliQ water (5/4/1, v/v/v), and for the purpose of further purification, Iatrobeads column chromatography was carried out again. The Iatrobeads activated beforehand with CHCl₃/ MeOH/ MilliQ water (5/4/1, v/v/v) were used.

The sample was applied to latrobeads with 25 ml of bed volume, and 75 ml of CHCl₃/ MeOH/ MilliQ water (5/4/1, v/v/v) was passed through. At this time, the eluate was fractionated into 0.25 ml fractions and 300 fractions were collected by using a fraction collector. These fractions were applied to an HPTLC plate, developed with CHCl₃/ MeOH/ 12 mM MgCl₂ (5/4/1, v/v/v), and mapping was carried out.

As a result, the target lipid molecule was not eluted with CHCl₃/ MeOH/MilliQ water (5/4/1, v/v/v), therefore, subsequently, 25 ml of CHCl₃/ MeOH/MilliQ water (40/50/9, v/v/v) was passed through, and the eluate was fractionated into 0.25 ml fractions and 100 fractions were collected.

These fractions were applied to an HPTLC plate, and developed over 20 cm with CHCl₃/ MeOH/ 12 mM MgCl₂ (5/4/1, v/v/v), and the separated 5 bands were scraped off and collected by using a surgical knife. The respective bands were extracted with 40 ml of CHCl₃/ MeOH/ MilliQ water (30/60/8, v/v/v), and sonicated for 5 minutes. Thereafter, in order to remove silica gel, the mixture was filtered through a cotton plug, and the solvent was removed. The residue was dissolved in 1 ml of CHCl₃/ MeOH (8/2, v/v), and Iatrobeads column chromatography was carried out. Iatrobeads was activated with CHCl₃/ MeOH (8/2, v/v), and bed volume was set to 1 ml. After applying the sample thereto, 15 ml of CHCl₃/ MeOH (8/2, v/v), then 20 ml of CHCl₃/ MeOH/ MilliQ water (30/60/8, v/v/v) were passed through. The solvent of the fractions eluted with CHCl₃/ MeOH/ MilliQ water (30/60/8, v/v/v) was removed, and the residue was dissolved in 200 µl of CHCl₃/ MeOH (1/1, v/v). The obtained fractions were applied to an HPTLC plate, developed with the developing solvent, CHCl₃/MeOH/ 12 mM MgCl₂ (5/4/1, v/v/v), and then color was developed with an orcinol-H₂SO₄ reagent.

These fractions were named AM 1 to 5 and used as the final purification samples. With regard to AM 1 to 5, the binding specificity to type A and type B influenza viruses was compared with that of a standard glycolipid, sialylparagloboside.

### II. Results

The results of performing the first-round Iatrobeads column chromatography for the fractions of di- or higher sialo are shown in Fig. 5.

The eluted fractions were developed on a TLC plate, and then the color was developed with an orcinol-H₂SO₄ reagent that is a reagent for detecting sugar. With regard to the obtained fractions 1 to 7, reactivity with viruses was investigated, and the results are shown in Fig. 6.

A/Aichi/2/68 (Fig. 6c) strongly reacted with fractions 4 and 5, and A/Memphis/1/71 (Fig. 6d) particularly strongly reacted with fractions 4, 5 and 6. Since it was found that the regions shown by the arrow particularly strongly reacted, fractions 4 to 6 that contained more of this region were pooled, and the second-round Iatrobeads column chromatography was carried out.

The obtained pattern is shown in Fig. 7. Particularly, the regions indicated by the arrow contained more of the target glycolipid, and therefore, fractions 5 and 6 were collected and further purified.

Fractions 5 and 6 were purified separately, and only fraction 6 was developed over 20 cm by using an HPTLC plate. As a result, it was demonstrated that a band that was seemed to be broad was a mixture of at least five glycolipids. Accordingly, these 5 bands were scraped off and collected, and after removing silica gel, color development indicating sugar was investigated. Fig. 8 shows the obtained pattern. These 5 fractions were named AM 1 to 5, used as the final purification samples, and the binding specificity to type A and type B influenza viruses was compared with that of a standard glycolipid, sialylparagloboside.

With regard to AM 1 to 5, investigation was performed with the use of A/Aichi/2/68, A/PR/8/34 and A/Memphis/1/71 for type A. A/Aichi/2/6 that reacts with the sugar chain structure of Neu5Ac **α** 2-3Gal and Neu5Ac α 2-6Gal linkages to the same degree reacted with all the lipid molecules of AM1 to 5, and strongly reacted at the band which is observed at the mobility indicated by the arrow in AM1, and in AM 3 and 4 (Fig. 9c).

In addition, in Fig. 9, mass spectrometry could be carried out by the method described later in Example 3. In the cases of AM 1, 3, and 4, the same amount as that of α 2-3 sialylparagloboside and α 2-6 sialylparagloboside which are standard glycolipids was applied. Comparing AM1 with the standard glycolipids, the band indicated by the arrow in AM1 shows that AM1 reacted slightly more strongly than α 2-3 sialylparagloboside. AM 3 and 4 bound to α 2-6 sialylparagloboside at substantially the same strength.

On the other hand, A/PR/8/34 (Fig. 9d) that binds only to the sugar chain structure of Neu5Ac α 2-3Gal linkage strongly reacted with the band in AM1 indicated by the arrow, in the same manner as A/Aichi/2/68 (Fig. 9c). Therefore, it was indicated that AM1 has a very strong binding ability to an influenza virus compared with α 2-3 sialylparagloboside. In addition, it is different from A/Aichi/2/68 (Fig. 9c) in that it did not react with the bands observed at the upper region of mobility in AM 1 to 5. Further, A/Memphis/1/71 (Fig. 9e) that binds only to the sugar chain structure of Neu5Ac α 2-6Gal linkage shows a similar pattern to that of A/Aichi/2/68 (Fig. 9c).

AM1 reacted in the same manner to A/Aichi/2/68 and A/PR/8/34, and its reactivity was slightly weaker than that of α 2-6 sialylparagloboside. AM 3 and 4 reacted to the same degree as, or more than α 2-6 sialylparagloboside. From these results, the bands indicated by the arrow in AM1 strongly reacted with three viruses in the same way, and it was found that its reactivity is the same as, or stronger than that of the standard glycolipid.

In addition, with regard to AM1 to 5, the results of investigating the reactivity with type B influenza viruses are shown in Fig. 10. B/Gifu/2/73 (Fig. 10c) that binds to the sugar chain structure of Neu5Ac α 2-3Gal and Neu5Ac α 2-3Gal linkages to the same degree reacted with all the fractions 1 to 5. It was demonstrated that it also reacts with the band indicated by the arrow in AM1 in the same manner as three types of type A viruses. AM1 reacted more strongly than the standard glycolipids, α 2-3 and α 2-6 sialylparaglobosides. In addition, it did not react with the bands observed at the upper region of mobility in AM 1 to 3.

### Example 3 Structural analysis of AM 1, 3 and 4 by mass spectrometry

To analyze the structures of AM 1, 3 and 4, which could be subjected to mass spectrometry among AM 1 to 5, ESI (electrospray ionization)-FTMS (Fourier transform ion resonance mass spectrometer) assay and SIMS (secondary ion mass spectroscopy)-FTMS assay (BioAPEX (Bruker instruments) for both) were carried out.

### I. Experiments

### (1) ESI-FTMS

Each sample of AM 1, 3 and 4 was dissolved in methanol (3 pmol/µl), and spray was carried out at the flow rate of 1 µl/min by using a microsyringe pump. After adjusting an ESI high voltage controller with a cylinder, an endoplate, a capillary and the like, or a spray nozzle, measurement was carried out in the negative-ion mode.

### (2) SIMS-FTMS

Samples were dissolved in CHCl3/MeOH (2/1, v/v). A given amount (300 pmol) thereof was taken out and mixed with a matrix (triethanolamine), then measurement was carried out at the accelerating voltage of 10 kV in the negative-ion mode.

### II. Results

An m/Z of 2195 [M+Na-2H]- that is considered to be an ion peak associated with the molecular weight and an m/z of 1882 [M-NeuAc-H]- in which one sialic acid [-NeuAc-Na] was detached from the above molecule were confirmed. Further, an m/z of 290 [NeuAc]- and an m/z of 308 [NeuAc]- that are a peak associated with sialic acid were observed in a low molecular region. In addition, an m/z of 1086 [M-2H]2- was observed in a divalent ion region, and the molecular weight calculated from this agrees with the mass number of the ion peak associated with the molecular weight described above. Therefore, a possibility was suggested that two sialic acids are included.

With regard to the linkages of the two sialic acids, an analysis was carried out with reference to the spectra of GD1a and GD1b. As a result, an m/z of 581 and an m/z of 603, which were observed in NeuAc-NeuAc which is a component of GD1b, were not observed in AM1, therefore, it is considered that these sialic acids are bound to a neutral sugar separately. In addition, an ion peak of m/Z of 2195 associated with the molecular weight indicated a combination including a ceramide (LCB 18:1 - FA 16:0), 6 neutral sugars (4 hexoses, 2 hexamines), 2 sialic acids and one Na.

Further, an m/z of 536 (LCB 18:1 - FA 16:0) corresponding to a ceramide was observed from the measurement of SIMS-FTMS. In addition, an m/z of 698 and an m/z of 860 corresponding to the peaks of this ceramide to which a hexose is added one by one, an m/z of 1063 corresponding to the peak indicating that a hexosamine has also been added, and an m/z of 1225 corresponding to the peak indicating that another hexose has been added were confirmed. From these results, it was indicated that AM1 has a lactosamine skeleton.

From the above, from chorioallantoic membrane of embryonated hen eggs that is used as a host for an influenza virus, isolation of a molecule that becomes a receptor candidate was successful. In addition, it was confirmed that this receptor molecule was represented by the following formula (I): 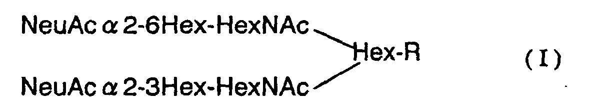 (wherein NeuAc represents *N*-acetylneuraminic acid in which the hydroxyl group, the carboxyl group and the amido group may be chemically modified with a halogen group, an alkyl group or an acyl group, either the same group or separate groups, Hex represents hexose, HexNAc represents *N*-acetylhexosamine and R represents a substrate selected from among a hydrogen atom, a hydrocarbon chain, a sugar chain, a lipid, a protein and a synthetic polymer, R may have a substituent) by a structural analysis.

### Example 4 Investigation of binding ability to various influenza viruses

### I. Experimental method

### (1) Materials

As an influenza virus strain, the ones shown in Table 5 were used.

**Table 5**

| Virus type | Serotype | Binding specificity to NeuAc |
|---|---|---|
| A/PR/8/34 | H1N1 | α 2-6 << α 2-3 |
| A/Aichi/2/68 | H3N2 | α 2-6 > α 2-3 |
| A/Memphis/1/71 | H3N2 | α 2-6 >> α 2-3 |

In the table, the binding specificity to NeuAc is based on the results of examining the reactivity of each virus strain with a standard glycolipid, sialylparagloboside, with the use of the foregoing TLC/virus-binding assay established by the inventors of this application.

As a standard glycolipid, the ones shown below were used.
- GM3

   Neu5Ac α 2-3Gal β 1-4Glcβ 1-1' Cer
- GSC-273 (synthetic)

   Neu5Ac a 2-3Gal β 1-4GlcNAc β 1-3Gal β 1-4Glc β 1-0CH(C₁₄H₂₉)₂
- GSC-275 (synthetic)

   Neu5Ac α 2-6 Gal β 1-4GlcNAc β 1-3Gal β 1-4Glc β 1-0CH(C₁₄H₂₉)₂

In addition, as an anti-influenza virus antibody, the ones shown in the following Table 6 were used.

**Table 6**

| Virus type (serotype) | Primary antibody*¹ | Secondary antibody*² |
|---|---|---|
| A/PR/8/34 (H1N1) | anti-P-50 (x 1000)*³ | Protein A (x 1000) |
| A/Aichi/2/68 (H3N2) | anti-P-50 (x 1000) | Protein A (x 1000) |
| A/Memphis/1/71 (H3N2) | USA 1 (x 500) | Protein A (x 1000) |

| | | |
|---|---|---|
| *1: The primary antibody is a rabbit polyclonal antibody produced by the inventors. | | |
| *2: anti-rabbit IgG-HRP (purchased from Santa Cruz Biotechnology), Protein A (purchased from Cappel) | | |
| *3: The number in parentheses indicates dilution ratio used in the experiment. | | |

### (2) Purification of influenza virus

A 0.2 ml portion of virus suspension diluted with sterilized PBS (type A influenza virus was diluted so as to have hemagglutination activity (HAU) = 2^{-3 to} ⁴, and type B influenza virus was diluted so as to have the hemagglutination activity (HAU) = 2^{-1 to 0}) was inoculated into the chorioallantoic cavity of 11-day old embryonated hen eggs and incubated at 34°C for 48 hours.

After chorioallantoic fluid was recovered, the fluid was centrifuged at 4°C for 10 minutes at 4000 rpm (HITACHI SCR 20B, RPR9 rotor) to remove impurities. The supernatant was further centrifuged at 4°C for 3 hours at 8000 rpm, and the pellet was suspended in PBS and used as a crude virus solution. Subsequently, the crude virus solution was deposited in a layer on 50% glycerol - PBS solution, and centrifuged at 4°C for 2 hours at 38000 rpm (HITACHI CP65β, P40ST rotor).

The obtained pellet was suspended in PBS and centrifuged at 4°C for 1 hour at 15000 rpm. The obtained pellet was suspended in PBS again, 10-40% (w/v) continuous sucrose density gradient centrifugation was carried out at 4°C for 90 minutes at 20000 rpm (HITACHI CP65β, P28S rotor). The band of virus was collected, and by performing centrifugation at 4°C for 3 hours at 8000 rpm (HITACHI SCR 20B, RPR18 rotor), a purified virus was obtained.

The obtained virus was suspended in PBS and stored at -80°C.

### (3) Measurement of HAU

A 50 µl portion of the purified influenza virus diluted 2¹⁰-fold was serially diluted 2-fold with 50 µl of 0.01 % gelatin (nacalai tesque, Inc.) in PBS on a microplate (96 U-bottom wells, Falcon). A 50 µl portion of 0.5% (v/v) guinea pig erythrocytes in PBS was added, let stand at 4°C for 2 hours, then hemagglutination was monitored. HAU was expressed as the maximum dilution of the virus required for causing agglutination of guinea pig erythrocytes.

### (4) Preparation of disialo-glycolipid fraction from chorioallantoic membrane of embryonated hen eggs

Chorioallantoic membrane was collected from 120 embryonated hen eggs of 11-day old, washed with PBS, and freeze-dried. To the dried chorioallantoic membrane, 100 ml of acetone was added and sonication was carried out for 10 minutes. After the mixture was homogenized by using a homogenizer, it was stirred at room temperature for 3 hours, and the obtained extract was filtered, then the residue was dried. Thereafter, 660 ml of CHCl₃/ MeOH/ MilliQ water (5:5:1, v/v/v) was added to the residue, and sonication was carried out for 10 minutes.

After the mixture was stirred overnight, it was filtered, then 300 ml of CHCl₃/ MeOH/ MilliQ water (30:60:8, v/v/v) was added to the residue and stirred for 2 hours. The filtrate was combined with the previous filtrate, and the solvent was removed with an evaporator. To the dried solid, 50 ml of MeOH was added, and sonication was carried out to suspend it well. Thereto was added a NaOH solution so that the final concentration became 0.1 N, then hydrolysis was carried out at 37°C for 3.5 hours, and further at room temperature overnight. After AcOH was added so that the final concentration became 0.1 N, the solvent was removed. After 25 ml of MilliQ water was added to the dried solid, sonicated, then dialysis was carried out. After the dialysis, the freeze-dried sample was dissolved in 50 ml of CHCl₃/ MeOH/ MilliQ water (30:60:8, v/v/v), sonicated, and filtered.

To the residue, 20 ml of CHCl₃/ MeOH/ MilliQ water (30:60:8, v/v/v) was added, and the same procedure was carried out. The filtrates of the operations done twice were combined and used as the sample for DEAE-Sephadex A-25 column chromatography. DEAE-Sephadex A-25 was used after it was activated with CHCl₃/ MeOH/ 0.8 M NaOAc (30:60:8, v/v/v) and equilibrated with CHCl₃/MeOH/ MilliQ water (30:60:8, v/v/v). The sample was applied to DEAE-Sephadex A-25 with 100 ml of bed volume, and 3 L of CHCl₃/ MeOH/MilliQ water (30:60:8, v/v/v) was passed through, whereby a neutral lipid fraction was eluted.

Thereafter, 3.5 L of CHCl₃/ MeOH/ 0.05 M NaOAc (30:60:8, v/v/v) was passed through the column, whereby a monosialo lipid fraction was eluted. Further, 2 L of CHCl₃/ MeOH/ 0.8 M NaOAc (30:60:8, v/v/v) was passed through the column, whereby an acidic lipid fraction of di- or higher sialo was eluted. With regard to the respective fractions of monosialo and di- or higher sialo, the solvent was removed with an evaporator. To the fraction of monosialo, 20 ml of MilliQ water was added, and to the fraction of di- or higher sialo, 20 ml of MilliQ water was added, respectively, and dialysis was carried out. After the dialysis, the respective freeze-dried fractions were dissolved in 1000 µl of CHCl₃/MeOH (1:1, v/v). For both of the fractions, the binding ability to an influenza virus was investigated by a TLC/ virus-binding assay.

### (5) TLC/virus-binding assay

After monosialo and disialo fractions that had been prepared from chorioallantoic membrane of embryonated hen eggs by DEAE-Sephadex A-255 column chromatography and standard glycolipids were applied on a plastic TLC plate, the plate was developed with acetone, air-dried, and continuously developed in the same direction with CHCl₃/ MeOH/ 12 mM MgCl₂ (5:4:1, v/v/v) and air-dried.

The plate was shaken in a PBS solution containing 1% ovalbumin and 1% polyvinylpyrrolidone (PVP) (Tokyo Kasei Kogyo Co., Ltd) (hereinafter referred to as Solution A) at room temperature for 1 hour, and washed three times with chilled PBS at 4°C for 5 minutes. Thereto was added 4 mL of an influenza virus suspension which had been diluted with PBS so as to have HAU = 2⁷, and was shaken at 4°C overnight. Incidentally, to the virus (-), PBS was added instead of a virus suspension. After the plate was washed three times with chilled PBS at 4°C for 5 minutes, 4 mL of a primary antibody solution diluted with a PBS solution containing 3% PVP (hereinafter referred to as Solution B) was added, shaken at 4°C for 2 hours, and washed three times with chilled PBS at 4°C for 5 minutes. Thereafter, 4 mL of a secondary antibody solution diluted with Solution B was added, and shaken at 4°C for 2 hours. After the reaction, the plate was washed three times with chilled PBS at 4°C for 5 minutes, a coloring substrate solution (0.1 M citrate buffer pH 6.0, 5 mL; 0.11 M 4-chloro-1-naphthol in CH₃CN, 100 µL; 0.06 M DEPDA in CH₃CN, 100 µL; 30% H₂O₂, 5 µL) was added and shaken at room temperature. After color was developed, the plate was washed with purified water and air-dried.

On another plate which had been developed and air-dried, an orcinol-H₂SO₄ reagent was sprayed, heated to 110°C, and detection of sugar was carried out. In the same manner, on another plate, a resorcinol-HCl reagent was sprayed and heated, and then detection of sialic acid was carried out. Then, the content of components of each band was quantified by using a densitometer (SHIMADZU).

### II. Experimental results

The acidic lipid fractions (monosialo and disialo fractions) obtained from chorioallantoic membrane of embryonated hen eggs were applied on TLC and developed. Thereafter, the color was developed with a resorcinol-HCl reagent, and the abundance of components included in each fraction was measured. TLC patterns shown in Fig. 11a and Fig. 11b were observed. From the quantification by using a densitometer, an abundance ratio of each component was calculated (shown at both sides of the plate in Fig 11b). In the same manner, the plate on which each fraction was spotted and developed, was reacted with virus strains of A/PR/8/34 (H1N1), A/Aichi/2/68 (H3N2), A/Memphis/1/71 (H3N2), respectively (Figs. 11e to g).

As a result, it was found that the glycolipid with high polarity present in the region indicated by the arrow in the figures reacted with all the viruses in the same way.

The inventors of this application have already isolated a receptor candidate molecule from the disialo fraction, and from the above results, the mobility of the substance indicated by the arrow is extremely similar to that of the candidate molecule. Therefore, it was suggested that the substance is the receptor candidate molecule itself.

Furthermore, from the quantification of the plate with a resorcinol-HCl reagent by using a densitometer, it was demonstrated that the content of the receptor candidate molecule included in the disialo fraction prepared this time is 1% or less (ratio of sialic acid amount).

### Example 5 Inhibitory activity on infection with various influenza viruses

### I. Experimental method

Measurement of inhibitory activity on infection with influenza viruses by LDH assay

Into a 96-well culture microplate (Nunc), MDCK cells were inoculated at a density of 2 x 10⁴ cells/well, and cultured in 100 µL of MEM medium containing 10% FCS at 37°C for 20 to 24 hours in the presence of 5% CO₂.

Subsequently, the disialo fraction prepared from chorioallantoic membrane of embryonated hen eggs was diluted up to 50 µL with serum-free MEM medium so that the final concentration of the total amount of sialic acid became 0.1, 0.3, 1, 3 and 10 µM, respectively, and 50 µL of an influenza virus solution diluted with serum-free MEM medium and 50 µL of serum-free MEM medium without a virus solution were added as a control, and preincubation was carried out at 4°C for 1 hour.

After confirming that the cells were grown to a confluent monolayer, the cells were washed 3 times with 100 µL of serum-free MEM medium, 100 µL of the preincubated virus solution and serum-free MEM medium were added and a control, and let stand at 34.5°C for 4 hours in the presence of 5% CO₂ to infect with the virus.

After infection, the supernatant was removed, the cells were washed once with 100 µL of serum-free MEM medium, 100 µL of MEM medium containing 5 % FCS was added, and cultured at 34.5°C for 20 to 24 hours in the presence of 5% CO₂. To a new microplate (96 Flat-Bottom Maxisorp, Nunc), 10 µL of (5X) PBS was added, then 40 µL of supernatant of cells cultured for 20 to 24 hours was added and mixed. After the cells were incubated at 37°C for 5 minutes, 50 µL of an LDH assay reagent (Shino-test co.) was added and mixed, then reacted at 37°C for 10 minutes.

The reaction was stopped by adding 100 µL of 0.5 N HCl, the absorbance of each well was measured with a measurement wavelength of 550 nm and a reference wavelength of 415 nm by using a microplate reader (MTP-32, Corona). Cell damage is caused by virus infection, and as a result, LDH that is an intracellular enzyme is released into the culture supernatant. By measuring this, virus infectivity can be evaluated.

The relative value of LDH activity in the supernatant of the sample containing disialo fraction of each concentration when the LDH activity in the supernatant of the control sample (with only a virus without containing a test sample) was assigned to be 100% was used as an indication for an inhibitory activity.

### II. Experimental results

Preincubation of the disialo fraction obtained from chorioallantoic membrane with a virus solution was carried out, and the effects on the virus infectivity of MDCK cells were investigated by the measurement of LDH activity. Where the LDH activity when only a virus solution was added is 100%, the relative activities when the disialo fraction at each concentration was added are shown in Fig. 12.

The TCID₅₀ (50% of tissue culture infectious dose, 50% inhibitory concentration) of disialo fraction for A/PR/8/34 (H1N1) and A/Aichi/2/68 (H3N2) was 1 µM (concentration of sialic acid), and that for A/Memphis/1/71 and (H3N2) B/Lee/40 was 3 µM (concentration of sialic acid).

Here, compared with the foregoing results of TLC/virus binding assay (Fig.11), the glycolipid has the ability to bind to respective viruses in the same way; that is, the receptor substance is considered to be the actual inhibitory active substance in the disialo fraction, and it has been demonstrated that the amount of this substance is 1 to 10% or less of the total amount of sialic acids in the disialo fraction.

From the above, it was suggested that a branched sialo-sugar chain receptor glycolipid in a small amount, 0.1 to 0.3 µM or less can in fact exert an inhibitory effect on virus infection of MDCK cells.

The inhibitory effect (TCID₅₀) of a compound having a virus-binding activity on influenza virus infection, which has been reported to date, is in the order of 10 µM. Compared with this, it was demonstrated that the branched sialo-sugar chain receptor glycolipid included in the disialo fraction of chorioallantoic membrane of embryonated hen eggs shows an extremely high anti-influenza virus activity.

### Industrial Applicability

As described in detail above, by the invention of this application, a novel branched sialo-sugar molecule is provided. This compound can bind to a type A influenza virus and a type B influenza virus originating in any animals including human, therefore, it can respond to a variation in the host range of an influenza virus or a variation in antigenicity, and its usefulness as a drug or an adsorbent in a virus removal filter or the like capable of preventing the infection with any influenza viruses is high.

## Claims

1. A novel branched sialo-sugar molecule **characterized by** being represented by the following formula (I): 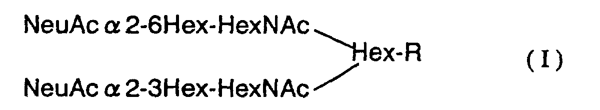 (wherein NeuAc represents *N*-acetylneuraminic acid in which the hydroxyl group, the carboxyl group and the amido group may be chemically modified with a halogen group, an alkyl group or an acyl group, either the same group or separate groups, Hex represents hexose, HexNAc represents *N*-acetylhexosamine and R represents a substrate selected from among a hydrogen atom, a hydrocarbon chain, a sugar chain, a lipid, a protein and a synthetic polymer, and R may have a substituent).

2. The novel branched sialo-sugar molecule according to claim 1, wherein the N-acetylneuraminic acid and hexose are linked by a natural O-glycoside linkage.

3. The novel branched sialo-sugar molecule according to claim 1, wherein the linkage between *N*-acetylneuraminic acid and hexose is a chemically converted linkage.

4. The novel branched sialo-sugar molecule according to claim 3, wherein the linkage form between *N*-acetylneuraminic acid and hexose is an S-glycoside linkage or a Se-glycoside linkage.

5. A novel branched sialo-sugar molecule **characterized by** being represented by the following formula (II): 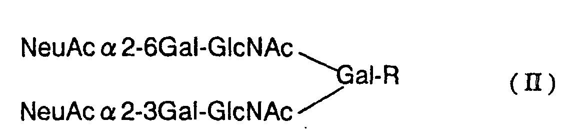 (wherein NeuAc represents *N*-acetylneuraminic acid in which the hydroxyl group, the carboxyl group and the amido group may be chemically modified with a halogen group, an alkyl group or an acyl group, either the same group or separate groups, Gal represents galactose, GlcNAc represents *N*-acetylglucosamine and R represents a substrate selected from among a hydrogen atom, a hydrocarbon chain, a sugar chain, a lipid, a protein and a synthetic polymer, and R may have a substituent).

6. A novel branched sialo-sugar molecule **characterized by** being represented by the following formula (III): 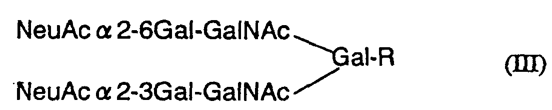 (wherein NeuAc represents *N*-acetylneuraminic acid in which the hydroxyl group, the carboxyl group and the amido group may be chemically modified with a halogen group, an alkyl group or an acyl group, either the same group or separate groups, Gal represents galactose, GalNAc represents *N*-acetylgalactosamine and R represents a substrate selected from among a hydrogen atom, a hydrocarbon chain, a sugar chain, a lipid, a protein and a synthetic polymer, and R may have a substituent).

7. The novel branched sialo-sugar molecule according to either claim 5 or 6, wherein the *N*-acetylneuraminic acid and galactose are linked by a natural O-glycoside linkage.

8. The novel branched sialo-sugar molecule according to either claim 5 or 6, wherein the linkage between *N*-acetylneuraminic acid and galactose is a chemically converted linkage.

9. The novel branched sialo-sugar molecule according to claim 8, wherein the linkage form between *N*-acetylneuraminic acid and galactose is an S-glycoside linkage or a Se-glycoside linkage.

10. An antiviral agent **characterized by** comprising at least the novel branched sialo-sugar molecule according to any one of claims 1 to 9 as an active ingredient.
